# EUROPEAN PATENT APPLICATION

(11) **EP 4 410 381 A1**
(43) Date of publication of application: **07.08.2024**
(21) Application number: 22875945.2
(22) Date of filing: 21.09.2022
(51) Int. Cl.: A61Q 1/00, A61Q 17/04, A61Q 19/00, A61Q 5/00, A61K 8/31, A61K 8/34

(54) **OIL FOR COSMETICS, AND COSMETIC CONTAINING SAME**

(30) Priority: 30.09.2021 JP 2021160330
(71) Applicant: NOF Corporation, Shibuya-ku Tokyo 150-6019 (JP)
(72) Inventor: SATO, Megumi, Kawasaki-shi Kanagawa 210-0865 (JP); SEKIGUCHI, Koji, Kawasaki-shi Kanagawa 210-0865 (JP)
(74) Representative: Mewburn Ellis LLP
(86) International application number: PCT/JP2022/035053
(87) International publication number: WO 2023/054075

(57) **Abstract**

The object is to provide a cosmetic oil agent capable of providing good affinity to the skin, powdery feeling with moisture and excellent rinsing property with water and to provide a cosmetic containing the same.

The cosmetic oil agent contains 97 to 99.9 mass% of (A) a straight-chain saturated hydrocarbon having a carbon number of 8 to 13, and 0.1 to 3 mass% of (B) a monovalent alcohol having a carbon number of 16 to 24.

## Description

### TECHNICAL FIELD

The present invention is to provide a cosmetic oil agent capable of providing good affinity to the skin, powdery and moisturizing feel and excellent rinsing property with water, and a cosmetic containing the same.

### BACKGROUND OF THE INVENTION

Until now, volatile oils and silicone oils have been blended in may products, such as skin care cosmetics, emulsified cosmetics, make-up cosmetics, anti-sunburn cosmetics, cleansing cosmetics or hair care cosmetics, as feel-improving agents.

Generally, as usability for the feel-improving agent, it is preferred to improve the affinity to the skin of a product, to suppress sticky feel of the product, and to further impart powdery feel (the feel of continuing sliding feel provided by a film formed on the skin after the application).

As volatile oil agents, such as cyclomethicone, light isoparaffin or isododecane, facilitate the affinity to the skin of a product and impart light usability thanks to the volatility, various volatile oil agents have been developed. For example, according to patent document 1, it is disclosed hydrocarbon mixture of hydrocarbon having a carbon number of 11 and hydrocarbon having a carbon number of 13.

However, as the volatile oil agent of patent document 1 has high volatility, water content is easily evaporated from a surface of a living body so that it is possibly provided powdery feeling without moisture. Further, in the case that the oil agent is blended in a high ratio as a feeling improver into a product and the product is applied on a face or hand and then rinsed, the poor ringing property of the oil agent with water may affect the product so that the product may possibly be sufficiently removed to result in unpleasant feeling.

Thus, according to patent document 2, it is disclosed paraffin mixture with excellent volatility, which may be suitably used as a cosmetic or cleansing oil agent applied on skin or hair and which contains paraffins including isoparaffin having a carbon number of 12 to 16 and 2, 2, 4, 6, 6-pentamethyl heptane.

### [Prior technical documents]

### [Patent documents]

[Patent document 1] Japanese patent publication No. 2010-530387A
[Patent document 2] WO 2013/118533 A1

### SUMMARY OF THE INVENTION

### [Object to be solved by the invention]

According to the mixture of patent document 2, it is obtained the paraffin mixture with good affinity to the skin and excellent moisturizing feel. However, as the moisturizing feel on the skin is improved, there is the problem that the powdery feeling is insufficient and the oil cannot be sufficiently removed to result in unpleasant feeling when the component is rinsed with water.

An object of the present invention is to provide a cosmetic oil agent, capable of providing good affinity to the skin, powdery feeling with moisture and excellent rinsing property with water, and to provide a cosmetic containing the same.

### [Solution for the object]

The present inventors have intensively researched the above items and found that the above problems can be solved by combining a specific straight-chain saturated hydrocarbon and a specific monovalent alcohol.

That is, the present invention is as follows.
(1) A cosmetic oil agent comprising:
   97 to 99.9 mass% of (A) a straight-chain saturated hydrocarbon having a carbon number of 8 to 13, and
   0.1 to 3 mass% of (B) a monovalent alcohol having a carbon number of 16 to 24.
(2) A cosmetic comprising 1 to 80 mass% of the cosmetic oil agent of (1).

### [Effects of the Invention]

According to the present invention, it is possible to obtain a cosmetic oil agent capable of providing good affinity to the skin, powdery feeling with moisture and excellent rinsing property with water, and to obtain a cosmetic containing the same.

### EMBODIMENTS FOR CARRYING OUT THE INVENTION

Embodiments of the present invention will be described below.

The present invention contains a component (A) and component (B), which will be described below in the order.

### [Component (A)]

As the component (A) (the straight-chain saturated hydrocarbon having a carbon number of 8 to 13) applied in the present invention, a kind of a raw material is not particularly limited as far as it can be conventionally blended in a cosmetic. The carbon number of the component (A) may preferably be 10 to 13, more preferably be 11 to 13 and most preferably be 12.

As specific examples of the component (A), n-octane, n-nonane, n-decane, n-undecane, n-dodecane, n-tridecane and the like are listed. One kind or two or more kinds of the components (A) may be used.

The content of the component (A) is made 97 to 99.9 mass%, provided that the total content of the cosmetic oil agent is made 100.0 mass%. In the case that the content of the component (A) is less than 97 mass%, the powdery feeling with moisture and rinsing property with water are insufficient. The content is thus made 97 mass% or higher, is preferably 97.5 mass% or higher, is more preferably 98 mass% or higher and is most preferably 98.5 mass% or higher. Further, in the case that the content of the component (A) exceeds 99.9 mass%, the affinity to the skin and powdery feeling with moisture may possibly be deteriorated. The content is thus made 99.9 mass% or lower, is preferably 99.7 mass% or lower and more preferably 99.5 mass% or lower.

Further, in the case that the total content of the cosmetic oil agent is made 100.0 mass%, on the viewpoint of irritation on skin and odor, the content of the straight-chain saturated hydrocarbon having a carbon number of 10 or lower is preferably 10 mass% or lower and more preferably 5 mass% or lower.

### [Component (B)]

As the monovalent alcohol having a carbon number of 16 to 24 of the component (B) applied in the present invention, a kind of a raw material is not particularly limited as far as it can be conventionally blended in a cosmetic. On the viewpoint of provision of the powdery feeling and rinsing property with water, the alcohol having a freezing point of 20°C or lower is preferred, the alcohol having a freezing point of 10°C or lower is more preferred, and the alcohol having a freezing point of 0°C or lower is most preferred. Further, the carbon number of the monovalent alcohol of the component (B) is preferably 16 to 22, more preferably 18 to 22 and most preferably 18 to 20.

As specific examples of the component (B), cetanol, cetearyl alcohol, stearyl alcohol, oleyl alcohol, behenyl alcohol, hexyl decanol, isostearyl alcohol, 2-octyl-1-dodecanol, 2-decyl-1-tetradecanol and the like are listed, isostearyl alcohol and 2-octyl-1-dodecanol are preferred and 2-octyl-1-dodecanol is more preferred. One kind or two or more kinds of the components (B) may be used.

The content of the component (B) is made 0.1 to 3 mass%, provided that the total content of the cosmetic oil agent is made 100 mass%. In the case that the content of the component (B) is less than 0.1 mass%, the affinity to the skin and rinsing property with water are insufficient. The content is thus made 0.1 mass% or higher, is more preferably 0.3 mass% or higher and most preferably 0.5 mass% or higher. Further, in the case that the content of the component (B) exceeds 3 mass%, the affinity to the skin, powdery feeling with moisture and rinsing property with water may possibly be deteriorated. The content is thus made 3 mass% or lower, is preferably 2.5 mass% or lower, is more preferably 2 mass% or lower and is most preferably 1.5 mass% or lower.

According to the present invention, the mass ratio ((A)/(B)) of the mass of the component (A) with respect to the mass of the component (B) may preferably 30 to 200, on the viewpoint of the affinity to the skin and powdery feeling. The affinity to the skin and powdery feeling with moisture can be further improved, in the case that the mass ratio (A)/(B) is made 30 or higher. The mass ratio is preferably 40 or higher, more preferably 50 or higher, and most preferably 80 or higher. Further, the affinity to the skin and rinsing property with water can be further improved in the case that the mass ratio (A)/(B) is made 200 or lower. The mass ratio is preferably 180 or lower, is more preferably 150 or lower and most preferably 120 or lower.

### (Cosmetics)

The cosmetic oil agent of the present invention can be applied in a cosmetic by blending it into the cosmetic as a raw material.

Although the applications of the inventive cosmetic oil agent of the present invention are not particularly limited, it may be suitably applied in, for example, a face cream, body cream, sun screen agent, moisturizing cream, hand cream, body cream, foot cream, massage cream, emulsion, foundation, oil beauty serum, massage oil, hair oil, cleansing oil and the like. Particularly, in the case that it is blended in the oil cosmetics such as the oil beauty serum, massage oil, hair oil, cleansing oil and the like, the affinity to the skin, powdery feeling and rinsing property with water can be further improved.

The cosmetic oil agent of the present invention is applied as a feeling improving agent in a cosmetic. The blending ratio of the inventive cosmetic oil agent may preferably be 1 to 80 mass%, more preferably be 1 to 60 mass% and most preferably be 1 to 50 mass%, provided that the content of the cosmetic is made 100 mass%. In the case that the blending ratio of the cosmetic oil agent is lower than 1 mass%, sufficient effect cannot be obtained.

Further, into the cosmetic of the present invention, other ingredients generally applied in cosmetics, such as oils and fats, higher fatty acids, higher alcohols, silicones, esters, surfactants, moisturizers, thickening agents, anti-oxidants, stabilizers, preservatives, pearling agents, pH adjusting agents, ultraviolet ray absorbers, hydrotropes, pigments, coloring agents, fungicides, antiinflammatory agents, astringents, refreshing agents, fragrances, plant essences and the like, may be optionally added. Further, as a solvent, optionally, water, ethanol, 1,3-butylene glycol, dipropylene glycol, propylene glycol, glycerin and the like may be applied.

### EXAMPLES

The present invention will be described in detail below, referring to the inventive and comparative examples.

The respective compositions shown in tables 1 and 2 were prepared as cosmetic oil agents. Further, the respective oil cosmetics shown in tables 3 and 4 were prepared as cosmetics with the cosmetic oil agents blended therein. Each of them was evaluated according to the following method and the results were shown in tables 1 to 4.

### (Evaluation items and evaluation standards)

### (1. Affinity to the skin)

20 females (with ages of 22 to 40) were selected as panelists, and the affinity to the skin was evaluated based on the following standard in the case that the cosmetic oil agent or oil cosmetic containing the same (about 1 g) was applied. Among them, the cosmetic oil agent and oil cosmetic were judged as having good affinity to the skin in the case that the result of "O" or "Ω" was obtained.
2 points: In the case that the affinity to the skin is very good after the application on the skin
1 point: In the case that the affinity to the skin is relatively good after the application on the skin
0 point: In the case that the affinity to the skin is relatively bad after the application on the skin

### (5-grade evaluation based on the total of the evaluation points)

Ω: The total of evaluation points is 35 points or higher.
O: The total of evaluation points is 30 points or higher and 34 points or lower.
Δ: The total of evaluation points is 20 points or higher and lower than 29 points.
×: The total of evaluation points is 19 points or lower.

### (2. Powdery feeling with moisture)

20 females (with ages of 22 to 40) were selected as panelists, and the powdery feeling with moisture was evaluated based on the following standard, in the case that the cosmetic oil agent or the oil cosmetic (about 1 g) containing the same was applied. The cosmetic oil agent or oil cosmetic was judged as having the powdery feeling with moisture in the case that the result of "O" or "Ω" was obtained.
2 points: In the case that the powdery feeling after the application on the skin is provided with moisture and very good
1 point: In the case that the powdery feeling after the application on the skin is provided with some moisture and good
0 point: In the case that the powdery feeling after the application on the skin lacks moisture feeling and is relatively bad

### (5-grade evaluation based on the total of evaluation points)

Ω: The total of evaluation points is 35 points or higher.
O: The total of evaluation points is 30 points or higher and 34 points or lower.
Δ: The total of evaluation points is 20 points or higher and lower than 29
×: The total of evaluation points is 19 points or lower.

### (3. Rinsing property with water)

20 females (with ages of 22 to 40) were selected as panelists, the cosmetic oil agent or oil cosmetic (about 3 g) containing the same was applied, and hands were then rinsed with cold water (5°C). The rinsing property at the time was evaluated based on the following standard. The cosmetic oil agent or oil cosmetic was judged as having the excellent rinsing property with water, in the case that the result of "O" or "Ω" was obtained.
2 points: The time duration required for completion of the rinsing is 15 seconds or shorter.
1 point: The time duration required for completion of the rinsing is shorter than 30 seconds.
0 point: The time duration required for completion of the rinsing is 30 seconds or longer.

### (5-grade evaluation based on the total of evaluation points)

Ω: The total of evaluation points is 35 points or higher.
O: The total of evaluation points is 30 points or higher and 34 points or lower.
Δ: The total of evaluation points is 20 points or higher and lower than 29 points
×: The total of evaluation points is 19 points or lower.

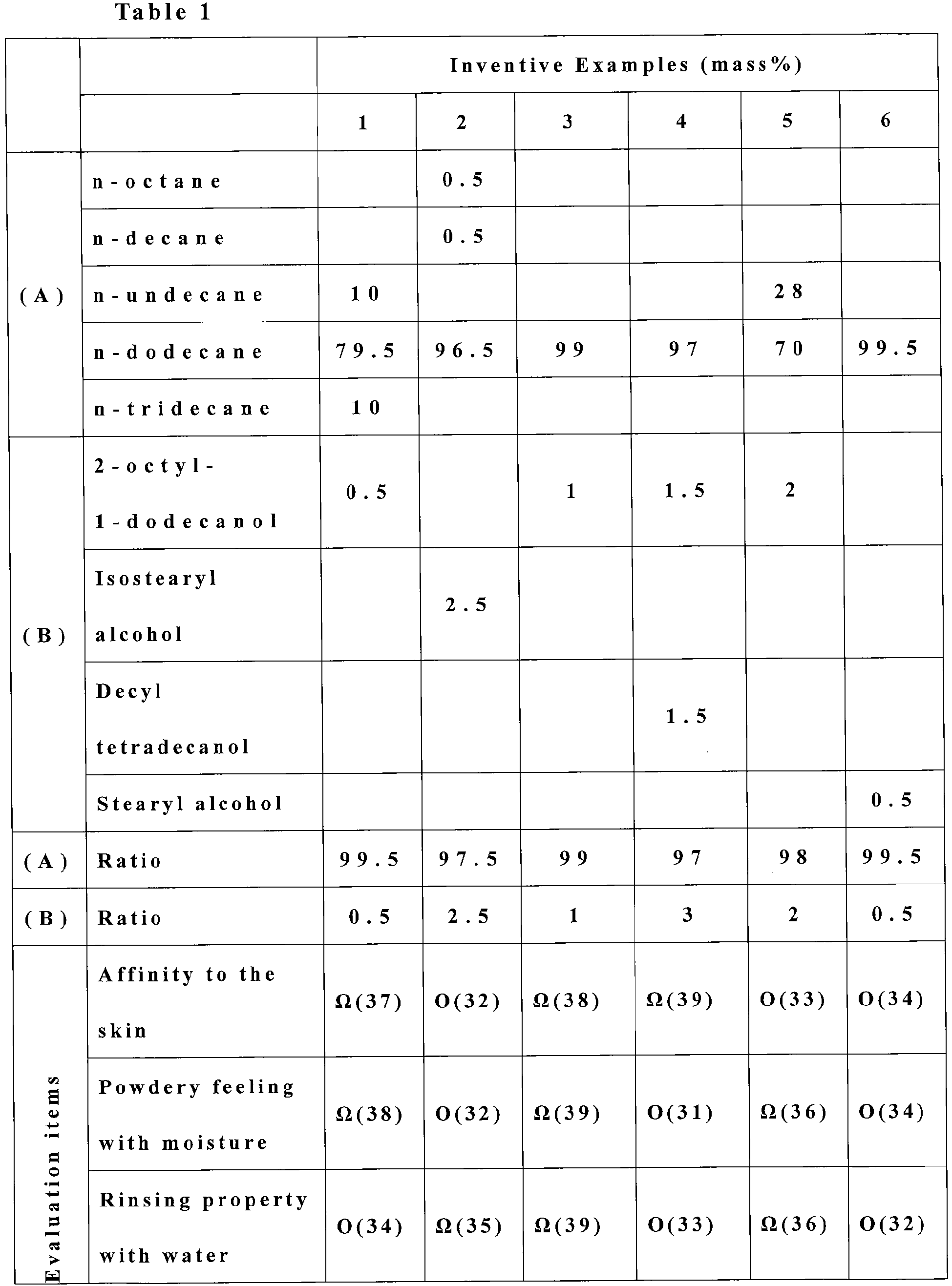

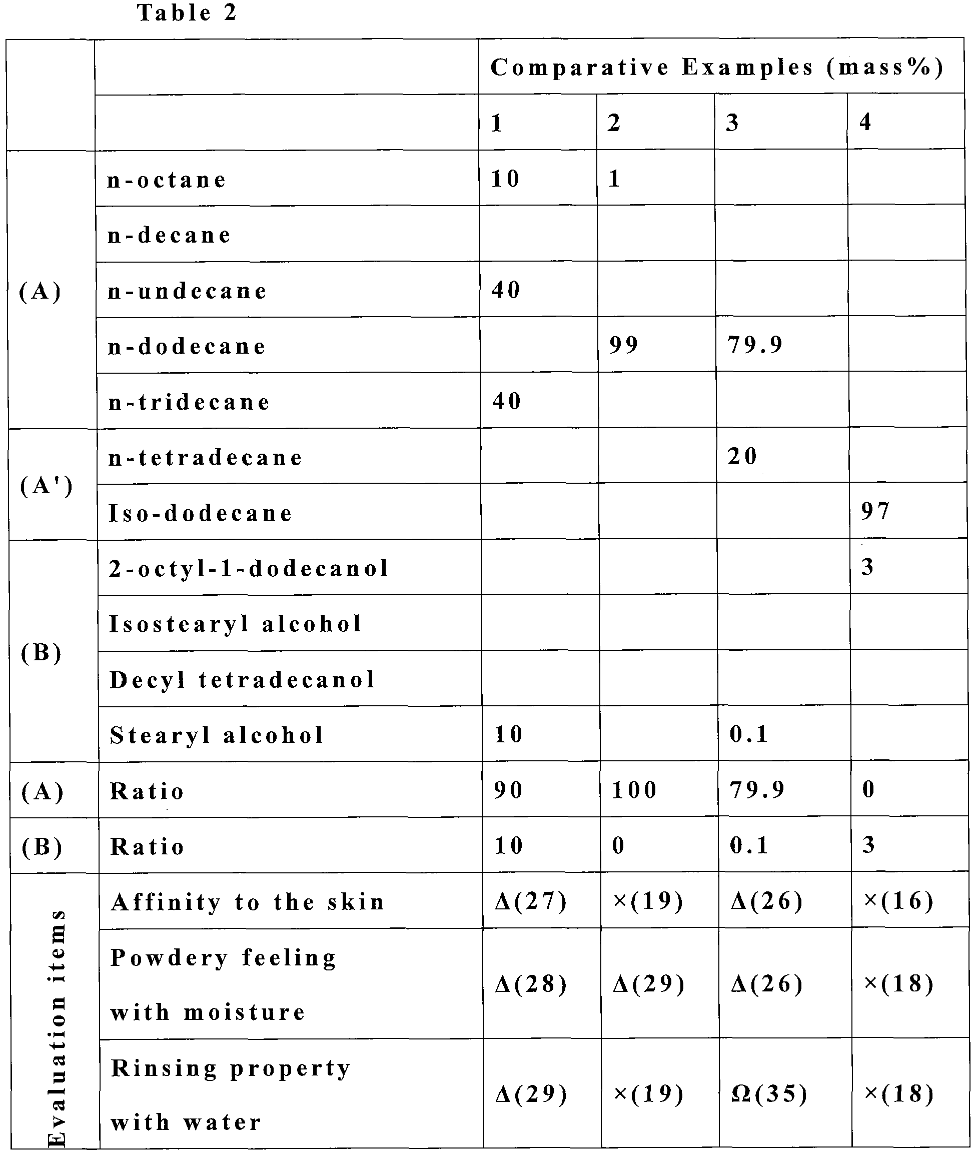

**Table 3**

| | Inventive examples (mass%) | | | | | | |
|---|---|---|---|---|---|---|---|
| | 7 | 8 | 9 | 10 | 11 | 12 | 13 |
| Inv. Ex. 1, cosmetic oil agent | 30 | | | | | | |
| Inv. Ex. 2, cosmetic oil agent | | 10 | | | | | |
| Inv. Ex. 3, cosmetic oil agent | | | 2 | 50 | | | |
| Inv. Ex. 4, cosmetic oil agent | | | | | 60 | | |
| Inv. Ex. 5, cosmetic oil agent | | | | | | 50 | |
| Inv. Ex. 6, cosmetic oil agent | | | | | | | 50 |
| Common added components | 70 | 90 | 98 | 50 | 40 | 50 | 50 |
| Total | 100 | | | | | | |
| Affinity to the skin | Ω(37) | O(31) | Ω(37) | Ω(38) | Ω(37) | O(33) | O(34) |
| Powdery feeling with moisture | Ω(37) | O(31) | Ω(35) | Ω(38) | O(30) | Ω(35) | O(33) |
| Rinsing property with water | O(34) | O(34) | Ω(35) | Ω(39) | O(30) | Ω(36) | O(32) |

| | | | | | | | |
|---|---|---|---|---|---|---|---|
| Common added components: Olive oil, ethyl hexyl palmitate and silicone oil are blended in 1:1:1 and added. | | | | | | | |

**Table 4**

| | Comparative Examples (mass%) | | | |
|---|---|---|---|---|
| | 5 | 6 | 7 | 8 |
| Cosmetic oil agent of Comparative example 1 | 50 | | | |
| Cosmetic oil agent of Comparative example 2 | | 50 | | |
| Cosmetic oil agent of Comparative example 3 | | | 50 | |
| Cosmetic oil agent of Comparative example 4 | | | | 50 |
| Common added components | 50 | 50 | 50 | 50 |
| Total | | | | |
| Affinity to the skin | Δ(26) | ×(18) | Δ(25) | ×(15) |
| Powdery feeling with moisture | Δ(27) | Δ(28) | Δ(25) | ×(17) |
| Rinsing property with water | Δ(29) | ×(19) | Ω(35) | ×(18) |

| | | | | |
|---|---|---|---|---|
| Common added components; Olive oil, ethyl hexyl palmitate and silicone oil were blended in a ratio of 1:1:1 and added. | | | | |

According to the inventive examples 1 to 13, the cosmetic oil agents composed of the inventive components and the cosmetics containing the cosmetic oil agents were good in the affinity to the skin, powdery feeling with moisture and rinsing property with water.

Further, according to the comparative examples 1 to 8, sufficient performance could not be obtained.

That is, according to the comparative examples 1 and 5, as the component (B) was blended in a high ratio, the affinity to the skin, powdery feeling with moisture and rinsing property with water were insufficient.

According to the comparative examples 2 and 6, as the component (B) was not blended, the affinity to the skin, powdery feeling with moisture and rinsing property with water were insufficient.

According to the comparative examples 3 and 7, as a part of the component (A) was replaced with the other component, the affinity to the skin and powdery feeling with moisture were insufficient.

According to the comparative examples 4 and 8, as the component (A) was replaced with the other component, the affinity to the skin, powdery feeling with moisture and rinsing property with water were insufficient.

### (Inventive example 14; Emulsion)

The respective components and additives of the composition described in the following table 5 are mixed to prepare an emulsion. It is obtained emulsified cosmetic (emulsion) excellent in the affinity to the skin, powdery feeling with moisture and rinsing property with water.

**Table 5**

| | Contents (mass%) |
|---|---|
| Cosmetic oil agent of inventive example 3 | 5% |
| Squalane | 2% |
| Dimethicone | 1% |
| Olive fruit oil | 2% |
| Ethyl hexyl palmitate | 2% |
| Glycerin | 5% |
| Butylene Glycol | 2% |
| Polyglyceryl-5 Stearate | 3% |
| PEG/PPG/polybutylene glycol-8/5/3 glycerin | 0.5% |
| Polyquaternium-51 | 0.1% |
| Sodium Hyaluronate (1% aqueous solution) | 0.01% |
| Dipotassium Glycyrrhizate | 1% |
| Phenoxy ethanol | 0.3% |
| Ethyl hexyl glycerin | 0.1% |
| Carbomer | 0.3% |
| Pottasium Hydroxide | Appropriate |
| Water | Residual part |
| Total | 100% |

### (Inventive example 15: Cream)

The components and additives of the composition described in the following table 6 are mixed to prepare a cream. It is obtained emulsified cosmetic (cream) excellent in the affinity to the skin, powdery feeling with moisture and rinsing property with water.

**Table 6**

| | Contents (mass%) |
|---|---|
| Cosmetic oil agent of inventive example 3 | 3% |
| Squalane | 2% |
| Olive fruit oil | 2% |
| Ethyl hexyl palmitate | 2% |
| Glycerin | 5% |
| Propylene glycol | 3% |
| Butylene glycol | 2% |
| Behenyl alcohol | 2% |
| Beeswax | 1% |
| Polyglyceryl-5 Laurate | 3% |
| PEG/PPG/polybutylene glycol-8/5/3 glycerin | 0.5% |
| Polyquaternium-51 | 0.1% |
| Sodium Hyaluronate (1% aqueous solution) | 0.01% |
| Tocopherol | 0.05% |
| Niacinamide | 3% |
| Phenoxy ethanol | 0.2% |
| Ethyl hexyl glycerin | 0.1% |
| Citric acid | Appropriate amount |
| Sodium citrate | Appropriate amount |
| Fragrance | 0.05% |
| Water | Residual part |
| Total | 100% |

### (Inventive example 16: Sun screen agent)

The components and additives of the composition described in the following table 7 are mixed to prepare a sun screen agent. It is obtained the sun screen agent excellent in the affinity to the skin, powdery feeling with moisture and rinsing property with water.

**Table 7**

| | Contents (mass%) |
|---|---|
| Cosmetic oil agent of inventive example 3 | 3% |
| Squalane | 2% |
| Dimethicone | 1% |
| Olive fruit oil | 2% |
| Ethyl hexyl palmitate | 2% |
| Disteardimonium Hectorite | 2% |
| (acrylate/alkyl acrylate (C10-30)) cross-polymer | 0.1% |
| Hydrogenated polyisobutene | 5% |
| Titanium oxide | 6% |
| Zinc oxide | 5% |
| Octyl methoxycinnamate | 2.8% |
| Diethylamino hydroxybenzoyl hexyl benzoate | 2.2% |
| PEG/PPG/polybuthylene glycol-8/5/3 glycerin | 0.5% |
| Polyquaternium-51 | 0.1% |
| Glycerin | 5% |
| 1,3-butylene glycol | 2% |
| Sodium hyaluronate (1% aqueous solution) | 0.01% |
| Phenoxy ethanol | 0.2% |
| Ethyl hexyl glycerin | 0.1% |
| Arginine | Appropriate amount |
| Water | Residual part |
| Total | 100% |

### (Inventive example 17: Point make remover)

The respective components and additives of the composition described in the following table 8 are mixed to prepare a point make remover. It is obtained the point make remover capable of providing good affinity to the skin, powdery feeling with moisture and excellent rinsing property with water.

**Table 8**

| | Contents (mass%) |
|---|---|
| Cosmetic oil agent of inventive example 3 | 10% |
| Cetyl ethyl hexanoate | 15% |
| Caprylic/Capric Triglyceride | 1.5% |
| Sodium chloride | 1% |
| Dipropylene glycol | 7% |
| Glycerin | 3% |
| Pentylene glycol | 1% |
| Olive fruit oil | 1% |
| 1,2-hexane diol | 0.3% |
| Butylene glycol | 1% |
| Disodium EDTA | 0.25% |
| Tocopherol | 0.05% |
| Water | Residual part |
| Total | 100% |

### (Inventive example 18: Oil cleansing)

The respective components and additives of the composition described in the following table 9 are mixed to prepare an oil cleansing. It is obtained the oil cleansing capable of providing good affinity to the skin, powdery feeling with moisture and excellent rinsing property with water.

**Table 9**

| | Contents (mass%) |
|---|---|
| Cosmetic oil agent of inventive example 3 | 20% |
| Cetyl ethyl hexanoate | 20% |
| Squalane | 5% |
| Olive fruit oil | 10% |
| Sorbeth Tetraisostearate | 15% |
| Polyglyceryl-10 diisostearate | 6% |
| Sorbitan oleate | 4% |
| Glyceryl laurate | 3% |
| Polysorbate 20 | 7% |
| Ethyl oleate | 6% |
| Tocopherol | 0.05% |
| Fragrance | 0.3% |
| Water | Residual Part |
| Total | 100% |

### (Inventive example 19: Hair oil)

The respective components and additives of the composition described in the following table 10 are mixed to prepare a hair oil. It is obtained the hair oil capable of providing good affinity to the skin, powdery feeling with moisture and rinsing property with water.

**Table 10**

| | Contents (mass%) |
|---|---|
| Cosmetic oil agent of inventive example 3 | 15% |
| Dimethicone | 65% |
| Olive fruit oil | 10% |
| Amino propyl dimethicone | 5% |
| Tocopherol | 0.05% |
| Fragrance | 0.3% |
| Water | Residual part |
| Total | 100% |

### (Inventive example 20: Treatment)

The respective components and additives of the composition described in the following table 11 are mixed to prepare a treatment. It is obtained the treatment providing good affinity to the skin, powdery feeling with moisture and excellent rinsing property with water.

**Table 11**

| | Contents (mass%) |
|---|---|
| Cosmetic oil agent of inventive example 3 | 5% |
| Behenyl trimethyl ammonium chloride | 1.5% |
| Stearamidopropyl dimethylamine | 0.5% |
| Squalane | 1% |
| Dimethicone | 5% |
| Panthenol | 0.5% |
| Aminopropyl dimethicone | 1% |
| Cetyl Alcohol | 5% |
| Glycerin | 5% |
| Citric acid | Appropriate Amount |
| Sodium citrate | Appropriate Amount |
| Phenoxy ethanol | 5% |
| Tocopherol | 0.05% |
| Fragrance | 0.3% |
| Water | Residual part |
| Total | 100% |

### (Inventive example 21: Mascara)

The respective components and additives of the composition described in the following table 12 are mixed to prepare a mascara. It is obtained the mascara capable of providing the good affinity to the skin, powdery feeling with moisture and rinsing property with water.

**Table 12**

| | Contents (mass%) |
|---|---|
| Cosmetic oil agent of inventive example 3 | 20% |
| Iron oxides | 15% |
| Microcrystalline wax | 10% |
| Candelilla Wax | 1.5% |
| Alcohol | 1% |
| Talc | 1% |
| Trimethylsiloxy silicate | 10% |
| Disteardimonium hectorite | 2% |
| Dextrin palmitate | 1% |
| Propyl paraben | 0.05% |
| Tocopherol | 0.05% |
| Hydrogenated polyisobutene | Residual part |
| Total | 100% |

### (Inventive example 22: Liquid foundation)

The respective components and additives of the composition described in the following table 13 are mixed to prepare a liquid foundation. It is obtained the liquid foundation capable of the good affinity to the skin, powdery feeling with moisture and excellent rinsing property with water.

**Table 13**

| | Contents (mass%) |
|---|---|
| Cosmetic oil agent of inventive example 3 | 15% |
| Dimethicone | 15% |
| Beeswax | 1% |
| Sodium hyaluronate | 0.01% |
| Polyquaternium-51 | 0.01% |
| Butylene glycol | 5% |
| Dipropylene glycol | 5% |
| Propane diol | 3% |
| Aluminum hydroxide | Appropriate amount |
| Stearic acid | Appropriate amount |
| Tocopherol | 0.05% |
| Phenoxy ethanol | 0.5% |
| PEG-9 Polydimethylsiloxyethyl Dimethicone | 1% |
| Talc | 4% |
| Titanium oxide | 8% |
| Iron oxide | 2% |
| Water | Residual part |
| Total | 100% |

### (Inventive example 23: Lipstick)

The respective components and additives of the composition described in the following table 14 are mixed to prepare a lipstick. It is obtained the lipstick capable of providing the good affinity to the skin, powdery feeling with moisture and excellent rinsing property with water.

**Table 14**

| | Contents (mass%) |
|---|---|
| Cosmetic oil agent of inventive example 3 | 10% |
| Hydrogenated polyisobutene | Residual p:art |
| Castor oil | 5% |
| Jojoba oil | 5% |
| Olive oil | 5% |
| Carnauba wax | 2% |
| Polyethylene wax | 9% |
| Silicone resin | 18% |
| Dimethyl polysiloxane (SH100) | 5% |
| Aluminum hydroxide | Appropriate amount |
| Red No.201 | 1% |
| Titanium oxide | 2% |
| Pearling pigment | 5% |
| Total | 100% |

## Claims

1. A cosmetic oil agent comprising:
97 to 99.9 mass% of (A) a straight-chain saturated hydrocarbon having a carbon number of 8 to 13, and
0.1 to 3 mass% of (B) a monovalent alcohol having a carbon number of 16 to 24.

2. A cosmetic comprising 1 to 80 mass% of the cosmetic oil agent of claim 1.
